# EUROPEAN PATENT APPLICATION

(11) **EP 4 752 160 A1**
(43) Date of publication of application: **03.06.2026**
(21) Application number: 24844367.3
(22) Date of filing: 24.05.2024
(51) Int. Cl.: C07K 16/28, C12N 15/62, C12N 15/13, C07K 19/00, A61K 39/00, C12N 5/10, A61K 48/00, A61P 35/02

(54) **HUMANIZED ANTI-CD19 SINGLE-CHAIN VARIABLE REGION FRAGMENT AND USE THEREOF**

(30) Priority: 24.07.2023 CN 202310908431
(71) Applicant: Triarm Inc., San Mateo, CA 94404 (US); Triarm Therapeutics (Shanghai) , Ltd., Shanghai 200120 (CN)
(72) Inventor: WENG, Jingjie, Shanghai 200120 (CN); LU, Jinhua, Shanghai 200120 (CN); ZHANG, Jay, Shanghai 200120 (CN); YANG, Hongchuan, Shanghai 200120 (CN); LU, Dexin, Shanghai 200120 (CN)
(74) Representative: Lavoix
(86) International application number: PCT/CN2024/095315
(87) International publication number: WO 2025/020672

(57) **Abstract**

Provided is a new humanized anti-CD 19 antibody. A humanized CAR-T cell is constructed on the basis of the antibody. The constructed humanized CAR-T cell has a killing effect and a tumor removing ability which are comparable to those of a CAR-T cell constructed using FMC63, and avoids safety risks such as an immune response caused by murine scFv. Therefore, the humanized CAR-T cell is more suitable for clinical treatment.

## Description

### TECHNICAL FIELD

The present invention relates to the field of antibody technology, and in particular to a humanized anti-CD19 single-chain variable region fragment and use thereof.

### BACKGROUND

Adoptive cellular immunotherapy (ACCT) has achieved remarkable success in recent years. With the US Food and Drug Administration's approval of KYMRIAH (Novartis Pharmaceuticals Corporation), YESCARTA and TECARTUS (Kite Pharma, Inc.), BREYANZI (Juno Therapeutics, Inc.), ABECMA (Celgene Corporation), CARVYKTI (Janssen Biotech, Inc.) and the like for the treatment of relapsed and refractory hematologic malignancies including large B-cell lymphoma, mantle cell lymphoma, and multiple myeloma, it has become a new and reliable therapeutic strategy to prolong patients' survival and improve their quality of life. These FDA-approved ACCTs are all engineer autologous T cells to express chimeric antigen receptors (CARs), which target specific tumor antigens and stimulate and activate the adopted T cells to kill tumors, achieving anti-tumor effects. The chimeric antigen receptor structure consists of an extracellular antigen-binding domain connected to an intracellular secondary signaling domain and primary signaling domain via a hinge and transmembrane region. The extracellular antigen-binding domains of KYMRIAH, YESCARTA, TECARTUS, and BREYANZI are all mouse-derived single-chain variant fragments of anti-CD19. The extracellular antigen-binding domain of ABECMA is a mouse-derived single-chain variant fragment of anti-BCMA; and the extracellular antigen-binding domain of CARVYKTI is a single-domain antibody derived from alpaca. However, these non-human protein sequences, when exposed outside of T cells, increase the immunogenicity of these agents, potentially eliciting an immune response in the human body. This shortens the effectiveness of these adoptive T cell immunotherapies and raises safety concerns.

CAR-T cells with mouse-derived scFvs can induce humoral and cellular immune responses, including excessive expression of HAMA (human anti-mouse antibodies) and proliferation of CD8 T cells targeting the mouse scFv. This results in a short CAR-T half-life, poor efficacy, and safety concerns. If patients have previously received treatment with antibodies containing mouse sequences (such as Rituximab, infliximab, and Aciximab), the potential for anti-drug antibodies (ADA), whether related to or unrelated to the disease being treated by the CAR-T, increases the risk of mouse-derived CAR-T therapy. CAR-T cells based on non-mouse and non-human extracellular antigen-binding domains face the same challenges as mouse-derived scFv-based CAR-T cells.

Among patients with acute lymphoblastic leukemia who had relapsed or were unresponsive to the treatment of CD19-CAR-T cells containing mouse scFvs, 64% (7/11) achieved complete remission within one month after receiving CD19-CAR-T cells containing human scFvs. A clinical study using a humanized scFv-based CD19-CAR-T cell with 25% residual mouse sequence showed that 8 of 10 patients with relapsed or refractory acute lymphoblastic leukemia achieved complete remission for more than six months. HAMA levels peaked between three weeks and three months after infusion, and then declined sharply after three months. Compared to CD19-CAR-T cells based on mouse scFvs, HAMA induced by the aforesaid CAR-T cells still fluctuated three months after infusion, which exhibited a significantly lower immunogenicity. *In vitro* studies have shown that humanized scFv-based CD19-CAR-T cells have a weaker ability to induce IL-6 release from monocytes than mouse scFv-based CD19-CAR-T cells, thus reducing the risk of CRS in clinical use. In 2021, Myers et al. reported the treatment outcomes of 72 patients with relapsed or refractory B-cell acute lymphoblastic leukemia and two patients with B-cell lymphoblastic lymphoma treated with humanized scFv-based CD19-CAR-T cells. Among the 41 patients who had not previously received mouse CAR-T therapy, the one-month complete remission rate was 98%, while the one-month complete remission rate reached 64% in the 33 patients who had previously received mouse CAR-T therapy but had relapsed or had no response. The 12- and 24-month relapse-free survival rates for patients who had not previously received mouse CAR-T therapy were 84% and 74%, respectively, compared to 73% and 58% for patients who had previously received mouse CAR-T therapy. These studies have shown that humanized scFv-based CAR-T has significant advantages in efficacy and safety compared to mouse scFv-based CAR-T. Even patients who have received unsuccessful mouse CAR-T treatment may have a chance of being cured with humanized CAR-T.

Therefore, there is still a great demand for humanized CAR-T in this field.

### SUMMARY OF THE INVENTION

The purpose of the present invention is to provide a series of anti-CD19 single-chain variable region fragments with a degree of humanization greater than 90% (93.83%-99.56%), which can be integrated into chimeric antigen receptors to prepare chimeric antigen receptor T cells having good *in vivo* and *in vitro* anti-tumor (especially B cell lymphoma) functions.

In the first aspect of the present invention, it provides a humanized anti-CD19 antibody, comprising an antibody heavy chain variable region as set forth in any one of SEQ ID NOs: 1-4, and an antibody light chain variable region as set forth in any one of SEQ ID NOs: 5-7.

In another preferred embodiment, the antibody comprises an antibody light chain variable region and an antibody heavy chain variable region selected from the following groups:

| Antibody Number | Amino Acid Sequence of Heavy Chain Variable Region | Amino Acid Sequence of Light Chain Variable Region |
|---|---|---|
| VH4+VL3 | SEQ ID NO: 1 | SEQ ID NO: 5 |
| VH1+VL1 | SEQ ID NO: 2 | SEQ ID NO: 6 |
| VH2+VL1 | SEQ ID NO: 3 | SEQ ID NO: 6 |
| VH3+VL1 | SEQ ID NO: 4 | SEQ ID NO: 6 |
| VH4+VL1 | SEQ ID NO: 1 | SEQ ID NO: 6 |
| VH1+VL2 | SEQ ID NO: 2 | SEQ ID NO: 7 |
| VH2+VL2 | SEQ ID NO: 3 | SEQ ID NO: 7 |
| VH3+VL2 | SEQ ID NO: 4 | SEQ ID NO: 7 |
| VH4+VL2 | SEQ ID NO: 1 | SEQ ID NO: 7 |
| VH1+VL3 | SEQ ID NO: 2 | SEQ ID NO: 5 |
| VH2+VL3 | SEQ ID NO: 3 | SEQ ID NO: 5 |
| VH3+VL3 | SEQ ID NO: 4 | SEQ ID NO: 5. |

In another preferred embodiment, the antibody comprises the antibody heavy chain variable region as set forth in SEQ ID NO: 1, and the antibody light chain variable region as set forth in SEQ ID NO: 5; or
the antibody comprises the antibody heavy chain variable region as set forth in SEQ ID NO: 2, and the antibody light chain variable region as set forth in SEQ ID NO: 6; or
the antibody comprises an antibody heavy chain variable region as set forth in SEQ ID NO: 3, and an antibody light chain variable region as set forth in SEQ ID NO: 6.

In another preferred embodiment, the antibody comprises the antibody heavy chain variable region as set forth in SEQ ID NO: 1, and the antibody light chain variable region as set forth in SEQ ID NO: 5.

In another preferred embodiment, the antibody is a double-chain antibody, or a single-chain antibody.

In another preferred embodiment, the antibody is a full-length antibody protein or an antigen-binding fragment.

In another preferred embodiment, the antibody is a bispecific antibody or a multispecific antibody.

In another preferred embodiment, the antibody further comprises a linker peptide between the heavy chain variable region and the light chain variable region.

In another preferred embodiment, the structure of the antibody is shown in the following Formula A or Formula B:

V_{H}-V_{L} (A);

V_{L}-V_{H} (B)

wherein, V_{H} is the antibody heavy chain variable region; V_{L} is the antibody light chain variable region; "-" is a linker peptide or peptide bond; preferably, the structure is shown in Formula B.

In another preferred embodiment, the linker peptide is 1-5, preferably 1-4, and more preferably 3-4 consecutive sequences as set forth in SEQ ID NO: 11 (GGGGS), SEQ ID NO: 12 (GGGS), SEQ ID NO: 13 (SSSSG), SEQ ID NO: 14 (GSGSA), or SEQ ID NO: 15 (GGSGG).

In another preferred embodiment, the amino acid sequence of the antibody is set forth in any one of SEQ ID NOs: 8-10.

In the second aspect of the present invention, it provides a CD19-targeting antigen binding receptor (CAR), wherein the antigen binding domain of the CAR comprises the humanized anti-CD19 antibody of the first aspect of the present invention.

In another preferred embodiment, the structure of the CAR is shown in the following Formula I:

L-scFv-H-TM-C-CS (I)

wherein,
each "-" is independently a linker peptide or a peptide bond;
L is none or a signal peptide sequence;
scFv is the antigen-binding domain targeting CD19;
H is none or hinge region;
TM is a transmembrane domain;
C is a co-stimulatory signal molecule;
CS is an intracellular signaling sequence derived from CD3ζ.

In another preferred embodiment, the scFv has a structure as shown in Formula A or Formula B:

V_{H}-V_{L} (A);

V_{L}-V_{H} (B)

wherein, V_{H} is the antibody heavy chain variable region of the humanized anti-CD19 antibody; V_{L} is the antibody light chain variable region of the humanized anti-CD19 antibody; "-" is a linker peptide or peptide bond; preferably, a structure of Formula B.

In another preferred embodiment, the linker peptide is 1-5, preferably 1-4, and more preferably 3-4 consecutive sequences as set forth in SEQ ID NO: 11 (GGGGS), SEQ ID NO: 12 (GGGS), SEQ ID NO: 13 (SSSSG), SEQ ID NO: 14 (GSGSA), or SEQ ID NO: 15 (GGSGG).

In another preferred embodiment, the amino acid sequence of the V_{H} is set forth in SEQ ID NO: 1, the amino acid sequence of the V_{L} is set forth in SEQ ID NO: 5; or
the amino acid sequence of the V_{H} is set forth in SEQ ID NO: 2, the amino acid sequence of the V_{L} is set forth in SEQ ID NO: 6; or
the amino acid sequence of the V_{H} is set forth in SEQ ID NO: 3, the amino acid sequence of the V_{L} is set forth in SEQ ID NO: 6.

In another preferred embodiment, the scFv amino acid sequence is set forth in any one of SEQ ID NOs: 8-10.

In another preferred embodiment, the L is a signal peptide of a protein selected from the group consisting of CD8α, CD28, GM-CSF, IL-12, CD4, CD137, and a combination thereof.

In another preferred embodiment, the L is a signal peptide derived from CD8α.

In another preferred embodiment, the L has an amino acid sequence as set forth in SEQ ID NO: 16.

In another preferred embodiment, the H is a hinge region of a protein selected from the group consisting of CD8α, CD28, CD4, CD7, CD152, CD137, PD-1, and a combination thereof.

In another preferred embodiment, the H is independently a hinge region derived from CD8.

In another preferred embodiment, the H has an amino acid sequence as set forth in SEQ ID NO: 17.

In another preferred embodiment, the TM is a transmembrane region of a protein selected from the group consisting of CD28, CD3 epsilon, CD45, CD4, CD5, CD8, CD9, CD16, CD22, CD33, CD27, CD33, CD37, CD45, CD64, CD80, CD86, CD134, CD137, CD152, CD154, TCRα/β/γ/δ chain, PD-1, and a combination thereof.

In another preferred embodiment, the TM is each independently a transmembrane region derived from CD28.

In another preferred embodiment, the CD8-derived transmembrane region has an amino acid sequence as set forth in SEQ ID NO: 18.

In another preferred embodiment, the C is a co-stimulatory signal molecule of a protein selected from the group consisting of CD28, CD2, CD7, CD27, CD30, CD40, CD70, OX40 (CD134), 4-1BB (CD137), CD154 (CD40L), PD1, Dap10, CDS, ICAM-1, LFA-1 (CD11a/CD18), ICOS (CD278), NKG2D, GITR, TLR2, and a combination thereof.

In another preferred embodiment, the C is a costimulatory signal molecule derived from CD28.

In another preferred embodiment, the CD28-derived costimulatory signal molecule has an amino acid sequence as set forth in SEQ ID NO: 19 or 20.

In another preferred embodiment, the CS is derived from the intracellular signaling region of CD3ζ or FcyR.

In another preferred embodiment, the CS is derived from the intracellular signaling region of CD3ζ, which has an amino acid sequence as set forth in SEQ ID NO: 21.

In another preferred example, the CAR has an amino acid sequence as set forth in any one of SEQ ID NOs: 22-25.

In the third aspect of the present invention, it provides a nucleic acid molecule encoding the humanized anti-CD19 antibody of the first aspect of the present invention or the CD19-targeting CAR of the second aspect of the present invention.

In the fourth aspect of the present invention, it provides a vector, wherein the vector contains the nucleic acid molecule of the third aspect of the present invention.

In another preferred embodiment, the vector is selected from the group consisting of DNA, RNA, plasmid, lentiviral vector, adenoviral vector, retroviral vector, transposon, and a combination thereof.

In another preferred embodiment, the vector is a lentiviral vector.

In the fifth aspect of the present invention, it provides a host cell, which contains the vector of the fourth aspect of the present invention, or the exogenous nucleic acid molecule of the third aspect of the present invention being integrated into the chromosome, or expresses the humanized anti-CD19 antibody of the first aspect of the present invention or the CD19-targeting CAR of the second aspect of the present invention.

In the sixth aspect of the present invention, it provides an engineered immune cell, which contains the vector of the fourth aspect of the present invention, or the exogenous nucleic acid molecule of the third aspect of the present invention being integrated into the chromosome, or expresses the humanized anti-CD19 antibody of the first aspect of the present invention or the CD19-targeting CAR of the second aspect of the present invention.

In another preferred embodiment, the engineered immune cell is selected from the group consisting of:
(i) a chimeric antigen receptor T cell (CAR-T cell); or
(ii) a chimeric antigen receptor NK cell (CAR-NK cell).

In another preferred embodiment, the engineered immune cell includes an universal CAR-T cell.

In the seventh aspect of the present invention, it provides an immunoconjugate, comprising:
(a) the humanized anti-CD19 antibody of the first aspect of the present invention;
(b) a coupling moiety selected from the group consisting of a detectable label, a drug, a toxin, a cytokine, a radionuclide, and an enzyme.

In another preferred embodiment, the coupling moiety is a drug or a toxin.

In another preferred embodiment, the coupling moiety is a detectable label.

In another preferred embodiment, the coupling moiety is selected from: fluorescent or luminescent labels, radioactive labels, MRI (magnetic resonance imaging) or CT (electronic computer X-ray tomography technology) contrast agents, or enzymes capable of producing detectable products, radionuclides, biotoxins, cytokines (such as IL-2, etc.), antibodies, antibody Fc fragments, antibody scFv fragments, gold nanoparticles/nanorods, viral particles, liposomes, nanomagnetic particles, prodrug-activating enzymes (for example, DT-diaphorase (DTD) or biphenyl hydrolase-like protein (BPHL)), chemotherapeutic agents (for example, cisplatin) or any form of nanoparticles, etc.

In the eighth aspect of the present invention, it provides a kit comprising the humanized anti-CD19 antibody of the first aspect of the present invention or the immunoconjugate of the seventh aspect of the present invention.

In another preferred embodiment, the kit further comprises an instruction manual, which indicates that the kit is used for detecting CD19 molecules.

In the ninth aspect of the present invention, it provides a preparation containing the humanized anti-CD19 antibody of the first aspect of the present invention, or the immunoconjugate of the seventh aspect of the present invention, or the engineered immune cell of the sixth aspect of the present invention, and a pharmaceutically acceptable carrier.

In another preferred embodiment, the preparation is a liquid preparation.

In another preferred embodiment, the preparation is in the form of an injection.

In another preferred embodiment, the concentration of the engineered immune cells in the preparation is 1×10³ -1×10⁸ cells/ml, preferably 1×10⁴ -1×10⁷ cells/ml.

In the tenth aspect of the present invention, it provides a use of the humanized anti-CD19 antibody of the first aspect of the present invention, or the engineered immune cell of the sixth aspect of the present invention, or the immunoconjugate of the seventh aspect of the present invention for (1) preparing a drug or preparation for treating cancer or tumors; (2) preparing a reagent or kit for detecting CD19 molecules.

In another preferred embodiment, the tumor or cancer is a tumor or cancer with high expression of CD19.

In another preferred embodiment, the tumor or cancer is selected from the group consisting of hematological tumors, solid tumors, and a combination thereof.

In another preferred embodiment, the hematological tumor is selected from the group consisting of lymphoma, multiple myeloma (MM), acute myeloid leukemia (AML), chronic lymphocytic leukemia (CLL), acute lymphoblastic leukemia (ALL), and a combination thereof.

In another preferred embodiment, the solid tumor is selected from the group consisting of gastric cancer, peritoneal metastasis of gastric cancer, liver cancer, renal tumor, lung cancer, small intestine cancer, bone cancer, prostate cancer, colorectal cancer, breast cancer, large intestine cancer, cervical cancer, ovarian cancer, lymphoma, nasopharyngeal carcinoma, adrenal tumor, bladder tumor, non-small cell lung cancer (NSCLC), glioma, endometrial cancer, testicular cancer, colorectal cancer, urinary tract tumor, thyroid cancer, and a combination thereof.

In another preferred embodiment, the cancer or tumor is lymphoma.

In another preferred embodiment, the cancer is B-cell lymphoma.

In the eleventh aspect of the present invention, it provides a method for preparing the engineered immune cell of the sixth aspect of the present invention, which comprises the following steps:
transducing the nucleic acid molecule of the third aspect of the present invention or the vector of the fourth aspect of the present invention into immune cells, and culturing the immune cells under conditions suitable for antibody or CAR expression to obtain the engineered immune cells.

In another preferred embodiment, the immune cells are T cells or NK cells.

In the twelfth aspect of the present invention, it provides a method for detecting CD19 protein in a sample, which comprises the steps of:
(1) contacting a sample with the humanized anti-CD 19 antibody of the first aspect of the present invention or the immunoconjugate of the seventh aspect of the present invention;
(2) detecting whether an antigen-antibody complex is formed, wherein the formation of the complex indicates the presence of CD19 protein in the sample.

In another preferred embodiment, the method is an *in vitro* method.

In another preferred embodiment, the method is non-therapeutic and non-diagnostic.

In the thirteenth aspect of the present invention, it provides a method for treating cancers or tumors, which comprises a step of administering to a subject in need thereof a therapeutically effective amount of the humanized anti-CD19 antibody of the first aspect of the present invention, or the immunoconjugate of the seventh aspect of the present invention, or the engineered immune cell of the sixth aspect of the present invention, or the preparation of the ninth aspect of the present invention.

In another preferred embodiment, the subject includes a human or a non-human mammal.

In another preferred embodiment, the tumor or cancer is a tumor or cancer with high expression of CD19.

In another preferred embodiment, the tumor or cancer is selected from the group consisting of hematological tumors, solid tumors, and a combination thereof.

In another preferred embodiment, the hematological tumor is selected from the group consisting of lymphoma, multiple myeloma (MM), acute myeloid leukemia (AML), chronic lymphocytic leukemia (CLL), acute lymphoblastic leukemia (ALL), and a combination thereof.

In another preferred embodiment, the solid tumor is selected from the group consisting of gastric cancer, peritoneal metastasis of gastric cancer, liver cancer, renal tumor, lung cancer, small intestine cancer, bone cancer, prostate cancer, colorectal cancer, breast cancer, large intestine cancer, cervical cancer, ovarian cancer, lymphoma, nasopharyngeal carcinoma, adrenal tumor, bladder tumor, non-small cell lung cancer (NSCLC), brain glioma, endometrial cancer, testicular cancer, colorectal cancer, urinary tract tumor, thyroid cancer, and a combination thereof.

In another preferred embodiment, the cancer or tumor is lymphoma.

In another preferred embodiment, the cancer is B-cell lymphoma.

It should be understood that, within the scope of the present invention, the above-described technical features of the present invention and the technical features specifically described below (such as in the examples) can be combined with each other to form new or preferred technical solutions, which is not redundantly repeated one by one herein due to space limitation.

### DESCRIPTION OF DRAWINGS

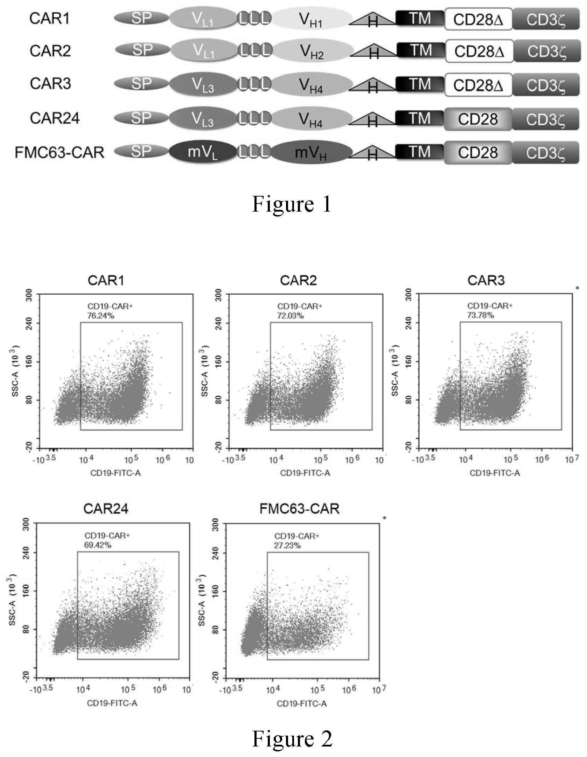

Figure 1: Schematic diagrams of CARs with different designs assembled with scFv in V_{L3}V_{H4} combination;
   wherein, SP: CD8α signal peptide; V_{L1}: Antibody light chain variable region 1; V_{L3}: Antibody light chain variable region 3; L: single linker (i.e., GGGGS); V_{H1}: Antibody heavy chain variable region 1; V_{H2}: Antibody heavy chain variable region 2; V_{H4}: antibody heavy chain variable region 4; H: CD8α hinge region; TM: CD8α transmembrane domain; CD28: CD28 intracellular domain; CD28Δ: CD28 intracellular region with Lck binding site mutation; CD3ζ: CD3zeta intracellular signaling domain.
Figure 2: Expression of CARs with different designs assembled with scFv in V_{L1}V_{H1}, V_{L1}V_{H2}, and V_{L3}V_{H4} combinations, delivered into in T cells via retroviral system.
Figure 3: Statistical results of the expression of CARs with different designs assembled with scFv in V_{L1}V_{H1}, V_{L1}V_{H2}, and V_{L3}V_{H4} combinations in T cells.
Figure 4: Comparison of yield between CAR-T cells of the present invention and FMC63 CAR-T cells.
Figure 5: Target cell killing capacity of CAR-T cells with different designs assembled with scFv in V_{L1}V_{H1}, V_{L1}V_{H2}, and V_{L3}V_{H4} combinations.
Figure 6: The *in vitro* killing capacity of humanized CD19 CAR-T cells against CD19-positive target cells is comparable to that of murine-derived FMC63 CD19 CAR-T cells.
Figure 7: The results of *in vivo* efficacy experiments for humanized CD19 CAR-T cells.

### DETAILED DESCRIPTION

Through extensive and in-depth research, the present inventors constructed a novel humanized anti-CD19 antibody for the first time, which was obtained by humanization of the FMC63 antibody. The present invention also provides CAR-T cells constructed based on this humanized antibody and related applications thereof. Compared to CAR-T cells constructed using FMC63, the CAR-T cells constructed in the present disclosure exhibit similar killing efficacy and tumor clearance capability, while avoiding safety concerns such as immune reactions caused by murine-derived scFv. The present invention was completed on this basis.

### Terms

To facilitate a better understanding of the present disclosure, certain terms are first defined. As used in the present application, unless otherwise expressly specified herein, each of the following terms shall have the meaning given below. Additional definitions are set forth throughout the application.

The term "about" may refer to a value or composition within an acceptable error range determined by a person of ordinary skill in the art, which will depend in part on how the value or composition is measured or determined.

The term "administering" or "administration" refers to physically introducing a product of the present invention into a subject using any of various methods and delivery systems known to those skilled in the art, including intravenous, intramuscular, subcutaneous, intraperitoneal, spinal, or other parenteral routes of administration, for example, by injection or infusion.

It should be understood that the amino acid names herein adopt the internationally recognized single-letter code. Their corresponding three-letter abbreviations are Ala(A), Arg(R), Asn(N), Asp(D), Cys(C), Gln(Q), Glu(E), Gly(G), His(H), Ile(I), Leu(L), Lys(K), Met(M), Phe(F), Pro(P), Ser(S), Thr(T), Trp(W), Tyr(Y), Val(V), respectively.

### Antibody

As used herein, the terms "antibody" or "immunoglobulin" are heterotetrameric glycoproteins of approximately 150,000 daltons with identical structural features, consisting of two identical light chains (L) and two identical heavy chains (H). Each light chain is linked to a heavy chain by one covalent disulfide bond, while the number of disulfide bonds varies between heavy chains of different immunoglobulin isotypes. Each heavy and light chain also has regularly spaced intrachain disulfide bonds. Each heavy chain has a variable region (VH) at one end, followed by multiple constant regions. Each light chain has a variable region (VL) at one end and a constant region at the other end; the constant region of the light chain is opposite to the first constant region of the heavy chain, and the variable region of the light chain is opposite to the variable region of the heavy chain. Specific amino acid residues form an interface between the variable regions of the light and heavy chains.

As used herein, the term "variable" refers to certain parts of the variable region in an antibody that are different in sequence, which form the binding and specificity of various specific antibodies to their specific antigens. However, variability is not evenly distributed throughout the variable region of an antibody. It is concentrated in three segments called complementarity determining regions (CDRs) or hypervariable regions in the light and heavy chain variable regions. The more conserved parts of the variable region are called framework regions (FRs). The variable regions of natural heavy and light chains each contain four FR regions, which are generally in a β-sheet configuration and are connected by three CDRs that form a connecting loop, and in some cases can form a partial β-sheet structure. The CDRs in each chain are closely together through the FR region and form the antigen-binding site of the antibody together with the CDRs of the other chain (see Kabat et al., NIH Publ. No. 91-3242, Volume 1, pages 647-669 (1991)). The constant regions do not directly participate in the binding of the antibody to the antigen, but they exhibit different effector functions, such as participating in the antibody-dependent cytotoxicity of the antibody.

The "light chain" of vertebrate antibodies (immunoglobulins) can be assigned to one of two distinct classes, called kappa and lambda (κ and λ), based on the amino acid sequence of their constant regions. Immunoglobulins can be divided into different classes based on the amino acid sequence of their heavy chain constant regions. There are five main classes of immunoglobulins: IgA, IgD, IgE, IgG, and IgM, some of which are further divided into subclasses (isotypes), such as IgG1, IgG2, IgG3, IgG4, IgA, and IgA2. The heavy chain constant regions corresponding to the different classes of immunoglobulins are called α, δ, ε, γ, and µ, respectively. The subunit structures and three-dimensional configurations of the different classes of immunoglobulins are well known in the art.

Generally, an antibody's antigen-binding properties are described by three specific regions located in the variable regions of the heavy and light chains, known as the variable regions (CDRs), to divide this fragment into four framework regions (FRs). The amino acid sequences of the four FRs are relatively conserved and do not directly participate in the binding reaction. These CDRs form a loop structure, spatially close to each other through the β-sheet formed by the FRs between them. The CDRs on the heavy chain and the corresponding CDRs on the light chain constitute the antibody's antigen-binding site. The amino acid sequences of antibodies of the same type can be compared to determine which amino acids constitute the FR or CDR regions.

The present invention includes not only complete antibodies, but also fragments of antibodies with immunological activity or fusion proteins formed by antibodies and other sequences. Therefore, the present invention also includes fragments, derivatives and analogs of the antibodies.

In the present invention, antibodies include murine, chimeric, humanized or fully human antibodies prepared using techniques well known to those skilled in the art. Recombinant antibodies, such as chimeric and humanized monoclonal antibodies, including human and non-human parts, can be obtained by standard DNA recombinant techniques, and they are all useful antibodies. A chimeric antibody is a molecule in which different parts are derived from different animal species, such as a chimeric antibody having a variable region from a mouse monoclonal antibody and a constant region from a human immunoglobulin (see, for example, U.S. Patent No. 4,816,567 and U.S. Patent No. 4,816,397, which are hereby incorporated by reference in their entirety). A humanized antibody refers to an antibody molecule derived from a non-human species, having one or more complementary determining regions (CDRs) derived from a non-human species and a framework region derived from a human immunoglobulin molecule (see U.S. Patent No. 5,585,089, which is hereby incorporated by reference in its entirety). These chimeric and humanized monoclonal antibodies can be prepared using DNA recombinant techniques well known in the art.

In the present invention, the antibodies can be monospecific, bispecific, trispecific, or more multispecific.

In the present invention, the antibodies of the present invention also include conservative variants thereof, which refer to polypeptides in which no more than 10, preferably no more than 8, more preferably no more than 5, and most preferably no more than 3 amino acids are substituted with amino acids having similar or similar properties, compared to the amino acid sequence of the antibodies of the present invention. These conservative variant polypeptides are preferably generated by amino acid substitution according to Table A.

**Table A**

| Original Residue | Representative Substitution | Preferred Substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

### Chimeric Antigen Receptor (CAR)

The chimeric antigen receptor (CAR) of the present invention includes an extracellular domain, a transmembrane domain, and an intracellular domain. The extracellular domain includes a target-specific binding element (also referred to as an antigen binding domain). The intracellular domain includes a costimulatory signaling region and a ζ chain portion. The costimulatory signaling region refers to a portion of the intracellular domain including costimulatory molecules. Costimulatory molecules are cell surface molecules required for the effective response of lymphocytes to antigens, rather than antigen receptors or their ligands.

Between the extracellular domain and the transmembrane domain of CAR, or between the cytoplasmic domain and the transmembrane domain of CAR, a linker can be incorporated. As used herein, the term "linker" generally refers to any oligopeptide or polypeptide that acts to connect the transmembrane domain to the extracellular domain or cytoplasmic domain of a polypeptide chain. The linker may include 0-300 amino acids, preferably 2 to 100 amino acids and most preferably 3 to 50 amino acids.

In a preferred embodiment of the present invention, the extracellular domain of the CAR provided by the present invention includes an antigen binding domain targeting CD19. When the CAR of the present invention is expressed in T cells, it is possible to perform antigen recognition based on antigen binding specificity. When it is combined with its associated antigen, it affects tumor cells, causing tumor cells to not grow, be prompted to die or otherwise be affected, and causes the patient's tumor load to shrink or be eliminated. The antigen binding domain is preferably fused with one or more intracellular domains from a costimulatory molecule and a ζ chain. Preferably, the antigen binding domain is fused with the intracellular domain of a combination of a CD28 signaling domain (including wild type and variants thereof, such as CD28 intracellular regions with Lck binding site mutations) and a CD3ζ signaling domain.

As used herein, both "antigen binding domain" and "single-chain antibody fragment" refer to Fab fragments, Fab' fragments, F(ab')₂ fragments, or single Fv fragments with antigen binding activity. Fv antibodies contain the variable region of the heavy chain and the variable region of the light chain of the antibody, but no constant region, and are the smallest antibody fragments with all antigen binding sites. Generally, Fv antibodies also contain a polypeptide linker between the VH and VL domains, and are capable of forming the structure required for antigen binding. The antigen binding domain is usually scFv (single-chain variable fragment). The size of scFv is generally 1/6 of a complete antibody. A single-chain antibody is preferably an amino acid chain sequence encoded by a nucleotide chain. As a preferred embodiment of the present invention, the antigen binding domain comprises an antibody that specifically recognizes CD19, preferably a single-chain antibody. Preferably, the scFv targeting CD19 of the present invention comprises an antibody heavy chain variable region as set forth in any one of SEQ ID NOs: 1-4, and an antibody light chain variable region as set forth in any one of SEQ ID NOs: 5-7.

For hinge region and transmembrane region (transmembrane domain), CAR can be designed to include a transmembrane domain fused to the extracellular domain of CAR. In one embodiment, a transmembrane domain naturally associated with one of the domains in CAR is used. In some examples, a transmembrane domain can be selected, or modified by amino acid substitution to avoid such a domain being bound to the transmembrane domain of the same or different surface membrane proteins, thereby minimizing the interaction with other members of the receptor complex.

### Chimeric antigen receptor T cells (CAR-T cells)

As used herein, the terms "CAR-T cells," "CAR-T," and "CAR-T cells of the present invention" include the CAR-T cells of the sixth aspect of the present invention. The CAR-T cells of the present invention can be used to treat tumors with high expression of CD19, such as multiple myeloma and lymphoma.

CAR-T cells have the following advantages over other T-cell-based treatments: (1) The action of CAR-T cells is not restricted by MHC; (2) Since many tumor cells express the same tumor antigens, once the CAR gene construction for a certain tumor antigen is completed, it can be widely used; (3) CAR can utilize both tumor protein antigens and glycolipid non-protein antigens, expanding the target range of tumor antigens; (4) The use of the patient's autologous cells reduces the risk of rejection; (5) CAR-T cells have immune memory function and can survive in the body for a long time.

### Chimeric antigen receptor NK cells (CAR-NK cells)

As used herein, the terms "CAR-NK cells," "CAR-NK," and "CAR-NK cells of the present invention" all refer to the CAR-NK cells encompassed by the sixth aspect of the present invention. The CAR-NK cells of the present invention can be used to treat tumors with high expression of CD19, such as multiple myeloma and lymphoma.

Natural killer (NK) cells are a major type of immune effector cell that protects the body from viral infection and tumor cell invasion through non-antigen-specific pathways. Engineered (genetically modified) NK cells may acquire new functions, including the ability to specifically recognize tumor antigens and enhanced anti-tumor cytotoxicity.

Compared with autologous CAR-T cells, CAR-NK cells have the following advantages: (1) They directly kill tumor cells by releasing perforin and granzymes, but have no killing effect on normal cells in the body; (2) They release very small amounts of cytokines, thereby reducing the risk of cytokine storms; (3) They are extremely easy to expand *in vitro* and develop into "ready-made" products. Other than that, it is similar to CAR-T cell therapy.

### Nucleic acid molecules and vectors

The nucleic acid sequence encoding the desired molecule can be obtained using recombinant methods known in the art, such as, for example, by screening libraries from cells expressing the gene, by obtaining the gene from a vector known to include the gene, or by directly isolating from cells and tissues containing the gene using standard techniques. Alternatively, the gene of interest can be produced synthetically.

The present invention also provides vectors into which the nucleic acid sequences or expression cassettes of the present invention are inserted. Vectors derived from retroviruses, such as lentiviruses, are suitable tools for achieving long-term gene transfer because they allow for long-term, stable integration of transgenes and their proliferation in daughter cells. Lentiviral vectors have advantages over vectors derived from oncogenic retroviruses, such as murine leukemia viruses, because they can transduce non-proliferating cells, such as hepatocytes. They also have the advantage of low immunogenicity.

In brief summary, the expression cassette or nucleic acid sequence of the present invention is generally operably linked to a promoter and incorporated into an expression vector. Such vectors are suitable for replication and integration into eukaryotic cells. Typical cloning vectors contain transcriptional and translational terminators, initiation sequences, and promoters that can be used to regulate expression of the desired nucleic acid sequence.

The expression constructs of the present invention can also be used for nucleic acid immunization and gene therapy using standard gene delivery protocols. Methods of gene delivery are known in the art. See, for example, U.S. Patent Nos. 5,399,346, 5,580,859, and 5,589,466, which are incorporated herein by reference in their entireties. In another embodiment, the present invention provides gene therapy vectors.

Nucleic acids can be cloned into many types of vectors. For example, nucleic acids can be cloned into such vectors, which include but are not limited to plasmids, phagemids, phage derivatives, animal viruses and cosmids. Specific vectors of interest include expression vectors, replication vectors, probe generation vectors and sequencing vectors.

Further, expression vector can be provided to cell in the form of viral vector. Viral vector technology is well known in the art and is described in, for example, Sambrook et al. (2001, Molecular Cloning: A Laboratory Manual, Cold Spring Harbor Laboratory, New York) and other virology and molecular biology manuals. The virus that can be used as a vector includes but is not limited to retrovirus, adenovirus, adeno-associated virus, herpes virus and lentivirus. Generally, a suitable vector comprises an origin of replication functional in at least one organism, a promoter sequence, a convenient restriction enzyme site and one or more selectable markers (for example, WO01/96584; WO01/29058; and U.S. Patent number 6,326,193).

Many virus-based systems have been developed for transferring genes into mammalian cells. For example, retroviruses provide a convenient platform for gene delivery systems. The selected gene can be inserted into a vector and packaged into retroviral particles using techniques known in the art. The recombinant virus can then be isolated and delivered to the subject's cells *in vivo* or *ex vivo.* Many retroviral systems are known in the art. In some embodiments, adenoviral vectors are used. Many adenoviral vectors are known in the art. In one embodiment, a lentiviral vector is used.

Additional promoter elements, such as enhancers, can regulate the frequency of transcription initiation. Typically, these are located in the 30-110 bp region upstream of the start site, although recently it has been shown that many promoters also contain functional elements downstream of the start site. The spacing between promoter elements is often flexible so that when an element is inverted or moved relative to another, promoter function is maintained. In the thymidine kinase (tk) promoter, the spacing between promoter elements can be increased by 50 bp before activity begins to decline. Depending on the promoter, it has been shown that individual elements can work together or independently to initiate transcription.

In order to evaluate the expression of the CAR polypeptide or its portion, the expression vector introduced into the cell may also include any one or both of a selectable marker gene or a reporter gene to facilitate identification and selection of expressing cells from a cell population that is sought to be transfected or infected by a viral vector. In other aspects, a selectable marker can be carried on a single DNA segment and used for co-transfection procedures. Both the selectable marker and reporter gene may be flanked by appropriate regulatory sequences to enable expression in host cells. Useful selectable markers include, for example, antibiotic resistance genes, such as neo and the like.

Methods for introducing genes into cells and expressing genes in cells are known in the art. In the content of expression vectors, the vectors can be readily introduced into host cells, such as mammalian, bacterial, yeast, or insect cells, by any method known in the art. For example, expression vectors can be transferred into host cells by physical, chemical, or biological means.

Physical methods for introducing polynucleotides into host cells include calcium phosphate precipitation, lipofection, particle bombardment, microinjection, electroporation, and the like. Methods for producing cells containing vectors and/or exogenous nucleic acids are well known in the art. See, for example, Sambrook et al. (2001, Molecular Cloning: A Laboratory Manual, Cold Spring Harbor Laboratory, New York).

Biological methods for introducing polynucleotides of interest into host cells include the use of DNA and RNA vectors. Viral vectors, particularly retroviral vectors, have become the most widely used method for inserting genes into mammalian, for example, human cells. Other viral vectors can be derived from lentiviruses, poxviruses, herpes simplex virus I, adenoviruses, and adeno-associated viruses, etc. See, for example, U.S. Patent Nos. 5,350,674 and 5,585,362.

Chemical means for introducing polynucleotides into host cells include colloidal dispersion systems, such as macromolecular complexes, nanocapsules, microspheres, beads, and lipid-based systems, including oil-in-water emulsions, micelles, mixed micelles, and liposomes. An exemplary colloidal system used as a delivery vehicle *in vitro* and *in vivo* is a liposome (e.g., an artificial membrane vesicle).

In the case of using non-viral delivery system, exemplary delivery means is liposome. Consider using lipid preparation, to introduce nucleic acid into host cell (*in vitro, ex vivo* or *in vivo*). On the other hand, this nucleic acid can be associated with lipid. The nucleic acid associated with lipid can be encapsulated in the aqueous interior of liposome, dispersed in the lipid bilayer of liposome, attached to liposome through the connecting molecule associated with liposome and oligonucleotide, trapped in liposome, compounded with liposome, dispersed in the solution comprising lipid, mixed with lipid, united with lipid, included in lipid as suspension, included in micelle or compounded with micelle, or in other manner. The lipid, lipid/DNA or lipid/expression vector associated with composition are not limited to any specific structure in solution. For example, they can be present in bilayer structure, as micelle or having "collapsed (collapsed) " structure. They can also be simply dispersed in solution, and may form aggregates of heterogeneous size or shape. Lipid is a fatty substance, which can be a naturally occurring or synthetic lipid. For example, lipids include fat droplets that occur naturally in the cytoplasm as well as compounds that contain long-chain aliphatic hydrocarbons and their derivatives such as fatty acids, alcohols, amines, amino alcohols, and aldehydes.

### Preparation

The present invention provides a preparation comprising the humanized anti-CD19 antibody of the first aspect of the present invention, or the immunoconjugate of the seventh aspect of the present invention, or the engineered immune cell of the sixth aspect of the present invention, and a pharmaceutically acceptable carrier. In a preferred embodiment of the present invention, the preparation comprises the CAR-T cells of the sixth aspect of the present invention, and a pharmaceutically acceptable carrier, diluent or excipient. In one embodiment, the preparation is a liquid preparation. Preferably, the preparation is an injection. Preferably, the concentration of the CAR-T cells in the preparation is 1×10³ -1×10⁸ cells/ml, preferably 1×10⁴ -1×10⁷ cells/ml.

In one embodiment, the preparation may include a buffer such as neutral buffered saline, sulfate buffered saline, or the like; a carbohydrate such as glucose, mannose, sucrose, or dextran, mannitol; a protein; a polypeptide or amino acid such as glycine; an antioxidant; a chelating agent such as EDTA or glutathione; an adjuvant (e.g., aluminum hydroxide); and a preservative. The preparation of the present invention is preferably formulated for intravenous administration.

### Therapeutic applications

The present invention includes therapeutic applications of cells (e.g., T cells) transduced with the retrovirus encoding the expression cassette of the present invention. The transduced T cells can target the tumor cell marker CD19, activate the T cells, elicit a T cell immune response, and thereby kill the tumor cells.

Therefore, the present invention also provides a method for stimulating a T cell-mediated immune response to a target cell population or tissue in a mammal, comprising the steps of administering the CAR-T cells of the present invention to the mammal.

In one embodiment, the present invention comprises a type of cell therapy in which a patient's autologous T cells (or those of an allogeneic donor) are isolated, activated, and genetically modified to produce CAR-T cells, which are then infused back into the same patient. This approach significantly reduces the risk of graft-versus-host disease, as antigens are recognized by T cells in an MHC-free manner. Furthermore, a single type of CAR-T cells can treat all cancers expressing that antigen. Unlike antibody therapies, CAR-T cells are able to replicate *in vivo,* resulting in long-term persistence that can lead to sustained tumor control.

In one embodiment, the CAR-T cells of the present invention can undergo robust *in vivo* T cell expansion and can sustain for an extended period of time. In addition, the CAR-mediated immune response can be part of an adoptive immunotherapy procedure, wherein the CAR-modified T cells induce an immune response specific to the antigen binding domain in the CAR. For example, anti-CD19 CAR-T cells induce a specific immune response against cells expressing CD19.

Although the data disclosed herein specifically disclose a lentiviral vector comprising an anti-CD19 scFv, hinge and transmembrane regions, and CD28 and CD3ζ signaling domains, the invention should be construed to encompass any number of variations in each of the construct's components.

Treatable cancers include tumors that are not vascularized or substantially not yet vascularized, and vascularized tumors. Cancer may include non-solid tumors (such as hematological tumors, including leukemia and lymphoma) or may include solid tumors. The types of cancer treated with the CAR of the present invention include but are not limited to cancer, blastoma and sarcoma, and certain leukemia or lymphoma, benign and malignant tumors and malignant tumors, such as sarcoma, cancer and melanoma. Also included are adult tumors/cancers and childhood tumors/cancers.

Hematological cancers are cancers of the blood or bone marrow. Examples of hematological (or hematogenous) cancers include leukemias, including acute leukemias (such as acute lymphocytic leukemia, acute myeloid leukemia, acute myeloid leukemia, and myeloblastic, promyelocytic, granulocytic-monocytic, monocytic, and erythroleukemias), chronic leukemias (such as chronic myeloid (granulocytic) leukemia, chronic myeloid leukemia, and chronic lymphocytic leukemia), polycythemia vera, lymphomas, Hodgkin's disease, non-Hodgkin's lymphoma (indolent and high-grade forms), multiple myeloma, Waldenstrom's macroglobulinemia, heavy chain disease, myelodysplastic syndrome, hairy cell leukemia, and myelodysplasia.

Solid tumors are abnormal masses of tissue that usually do not contain cysts or fluid areas. Solid tumors can be benign or malignant. Different types of solid tumors are named after the type of cells that form them (such as sarcomas, carcinomas, and lymphomas). Examples of solid tumors such as sarcomas and carcinomas include fibrosarcomas, myxosarcoma, liposarcoma, mesothelioma, lymphoid malignancies, pancreatic cancer, ovarian cancer, etc.

The CAR-modified T cells of the present invention can also be used as a vaccine type for *ex vivo* immunization and/or *in vivo* therapy of mammals. Preferably, the mammal is a human.

For *ex vivo* immunization, at least one of the following occurs *in vitro* prior to administering the cells into a mammal: i) expanding the cells, ii) introducing a nucleic acid encoding a CAR into the cells, and/or iii) cryopreserving the cells.

*Ex vivo* procedures are well known in the art and are discussed more fully below. Briefly, cells are isolated from mammals (preferably humans) and genetically modified (i.e., *in vitro* transduction or transfection) with a vector expressing the CAR disclosed herein. CAR-modified cells can be administered to a mammalian recipient to provide therapeutic benefits. The mammalian recipient can be a human, and the CAR-modified cells can be autologous relative to the recipient. Alternatively, the cells can be allogeneic, syngeneic, or xenogeneic relative to the recipient.

In addition to the use of cell-based vaccines for *ex vivo* immunization, the present invention also provides compositions and methods for *in vivo* immunization to elicit an immune response against an antigen in a patient.

The present invention provides a method for treating tumors, comprising administering a therapeutically effective amount of CAR-modified T cells of the present invention to a subject in need thereof.

The CAR-modified T cells of the present invention can be administered alone or as a pharmaceutical composition in combination with a diluent and/or with other components such as IL-2, IL-17 or other cytokines or cell groups. Briefly, the pharmaceutical composition of the present invention may include a target cell group as described herein, in combination with one or more pharmaceutically or physiologically acceptable carriers, diluents or excipients. Such compositions may include buffers such as neutral buffered saline, sulfate buffered saline, etc.; carbohydrates such as glucose, mannose, sucrose or dextran, mannitol; proteins; polypeptides or amino acids such as glycine; antioxidants; chelating agents such as EDTA or glutathione; adjuvants (e.g., aluminum hydroxide); and preservatives. The compositions of the present invention are preferably formulated for intravenous administration.

The pharmaceutical compositions of the present invention can be administered in a manner appropriate to the disease to be treated (or prevented). The amount and frequency of administration will be determined by factors such as the patient's condition, and the type and severity of the patient's disease, although appropriate dosages can be determined by clinical trials.

When an "immunologically effective amount," "anti-tumor effective amount," "tumor-suppressive effective amount," or "therapeutically effective amount" is indicated, the precise amount of the composition of the present invention to be administered can be determined by a physician, who takes into account individual differences in the patient's (subject's) age, weight, tumor size, degree of infection or metastasis, and condition. It can generally be stated that a pharmaceutical composition comprising the T cells described herein can be administered in a dose of 10⁴ to 10⁹ cells/kg body weight, preferably 10⁵ to 10⁶ cells/kg body weight (including all integer values within those ranges). The T cell composition can also be administered multiple times at these doses. The cells can be administered using infusion techniques well known in immunotherapy (see, for example, Rosenberg et al., New Eng. J. of Med. 319: 1676, 1988). The optimal dose and treatment regimen for a specific patient can be easily determined by a person skilled in the art of medicine by monitoring the patient's disease signs and adjusting the treatment accordingly.

Administration of the subject composition can be carried out in any convenient manner, including by spraying, injection, swallowing, infusion, implantation or transplantation. The compositions described herein can be administered to the patient subcutaneously, intradermally, intratumorally, intranodally, intraspinal, intramuscularly, by intravenous (i.v.) injection or intraperitoneally. In one embodiment, the T cell composition of the present invention is administered to the patient by intradermal or subcutaneous injection. In another embodiment, the T cell composition of the present invention is preferably administered by i.v. injection. The composition of the T cell can be injected directly into the tumor, lymph node or infection site.

In certain embodiments of the present invention, cells activated and expanded using the methods described herein or other methods known in the art for expanding T cells to therapeutic levels are administered to a patient in combination with (e.g., before, simultaneously, or after) any number of related treatment modalities, including, but not limited to, treatment with agents such as antiviral therapy, cidofovir and interleukin-2, cytarabine (also known as ARA-C), or natalizumab treatment for MS patients, or efavirenz treatment for psoriasis patients, or other treatments for PML patients. In further embodiments, the T cells of the present invention may be used in combination with chemotherapy, radiation, immunosuppressants such as cyclosporine, azathioprine, methotrexate, mycophenolate mofetil and FK506, antibodies, or other immunotherapeutic agents. In further embodiments, the cell compositions of the present invention are administered to a patient in combination with (e.g., before, simultaneously, or after) bone marrow transplantation, chemotherapy agents such as fludarabine, external beam radiation therapy (XRT), or cyclophosphamide. For example, in one embodiment, a subject may undergo standard treatment with high-dose chemotherapy followed by a peripheral blood stem cell transplant. In some embodiments, the subject receives an infusion of the expanded immune cells of the invention following transplantation. In an additional embodiment, the expanded cells are administered before or after surgery.

The dosage of the above treatments administered to a patient will vary with the precise nature of the condition being treated and the recipient of the treatment. Dosage ratios for human administration may be implemented according to practices accepted in the art. Typically, 1×10⁶ to 1×10¹⁰ modified T cells of the present invention are administered to the patient, for example, by intravenous infusion.

### The main advantages of the present invention include:

The CAR-T cells constructed using the humanized antibody scFv sequence designed and screened in the present invention are significantly superior to the CAR-T cells prepared based on FMC63 in terms of positive rate and total CAR-T cell yield. At the same time, they have similar *in vivo* and *in vitro* tumor killing abilities as the CAR-T constructed with FMC63, and can effectively avoid the humoral and cellular immune responses caused by the CAR-T constructed with mouse scFv, thereby effectively improving the safety and efficacy of the drug.

The present invention will be further illustrated below with reference to specific examples. It should be understood that these examples are intended to illustrate the present invention only and are not intended to limit the scope of the present invention. The conditions of the experimental methods not specifically indicated in the following examples are usually in accordance with conventional conditions as described in e.g., Sambrook et al., Molecular Cloning: A Laboratory Manual (New York: Cold Spring Harbor Laboratory Press, 1989), or according to the conditions recommended by the manufacturer. Unless otherwise stated, percentages and parts are calculated by weight.

### Example 1 Humanization Design

The FMC63 sequence was compared with a database of known human antibodies to identify the germline genes with the highest homology: IGHV4 for the heavy chain and IGKV1 for the light chain. Their framework regions were selected (using AbM to define CDRs and framework regions). Then, using computer prediction and simulation, mouse amino acids in the FMC63 antibody framework region that are important for antigen binding were retained through back mutation. Finally, the antigen-determining CDRs were replaced with the corresponding CDR sequences in the FMC63 antibody. The following humanized sequences were obtained:
Humanized anti-CD19 V_{H4} Sequence (SEQ ID NO: 1): 
QVQLQESGPGLVKPSETLSLTCTVSGVSLPDYGVSWIRQPPGKGLEWIGV
IWGSETTYYNPSLKSRVTISVDTSKNQVSLKLSSVTAADTAVYYCAKHYYYGG
SYAMDYWGQGTTVTVSS
Humanized anti-CD19 V_{H1} Sequence (SEQ ID NO: 2): 
EVQLQESGPGLVKPSETLSVTCTVSGVSLPDYGVSWIRQPPGKGLEWLG
VIWGSETTYYNSALKSRLTIIKDNSKSQVFLKMSSLTAADTAVYYCAKHYYYG
GSYAMDYWGQGTTVTVSS
Humanized anti-CD19 V_{H2} Sequence (SEQ ID NO: 3): 
QVQLQESGPGLVKPSETLSLTCTVSGVSLPDYGVSWIRQPPGKGLEWLGV
IWGSETTYYNSALKSRLTISKDNSKSQVFLKMSSVTAADTAVYYCAKHYYYG
GSYAMDYWGQGTTVTVSS
Humanized anti-CD19 V_{H3} Sequence (SEQ ID NO: 4): 
QVQLQESGPGLVKPSETLSLTCTVSGVSLPDYGVSWIRQPPGKGLEWLGV
IWGSETTYYNSALKSRVTISVDTSKNQVSLKLSSVTAADTAVYYCAKHYYYG
GSYAMDYWGQGTTVTVSS
Humanized anti-CD19 V_{L3} Sequence (SEQ ID NO: 5): 
DIQMTQSPSSLSASVGDRVTITCRASQDISKYLNWYQQKPGKAPKLLIYH
TSRLHSGVPSRFSGSGSGTDFTLTISSLQPEDFATYYCQQGNTLPYTFGGGTKL
EIK
Humanized anti-CD19 V_{L1} Sequence (SEQ ID NO: 6): 
DIQMTQSPSSLSASVGDRVTISCRASQDISKYLNWYQQKPGKAPKLLIYH
TSRLHSGVPSRFSGSGSGTDYTLTISSLQPEDFATYYCQQGNTLPYTFGGGTKL
EIK
Humanized anti-CD19 V_{L2} Sequence (SEQ ID NO: 7):

DIQMTQSPSSLSASVGDRVTITCRASQDISKYLNWYQQKPGKAPKLLIYH
TSRLHSGVPSRFSGSGSGTDYTLTISSLQPEDFATYYCQQGNTLPYTFGGGTKL
EIK

### Example 2 Construction of CAR-T cells containing humanized scFv

Furthermore, based on the heavy chain and light chain variable region sequences described in Example 1, scFv comprising the above variable regions was constructed:
> V_{L3}V_{H4} scFv (SEQ ID NO: 8) 
DIQMTQSPSSLSASVGDRVTITCRASQDISKYLNWYQQKPGKAPKLLIYH
TSRLHSGVPSRFSGSGSGTDFTLTISSLQPEDFATYYCQQGNTLPYTFGGGTKL
 
EIKGGGGSGGGGSGGGGSQVQLQESGPGLVKPSETLSLTCTVSGVSLPDYGVS
WIRQPPGKGLEWIGVIWGSETTYYNPSLKSRVTISVDTSKNQVSLKLSSVTAA
DTAVYYCAKHYYYGGSYAMDYWGQGTTVTVSS
> V_{L1}V_{H1} scFv (SEQ ID NO: 9) 
DIQMTQSPSSLSASVGDRVTISCRASQDISKYLNWYQQKPGKAPKLLIYH
TSRLHSGVPSRFSGSGSGTDYTLTISSLQPEDFATYYCQQGNTLPYTFGGGTKL
EIKGGGGSGGGGSGGGGSEVQLQESGPGLVKPSETLSVTCTVSGVSLPDYGVS
WIRQPPGKGLEWLGVIWGSETTYYNSALKSRLTIIKDNSKSQVFLKMSSLTAA
DTAVYYCAKHYYYGGSYAMDYWGQGTTVTVSS
> V_{L1}V_{H2} scFv(SEQ ID NO: 10)

DIQMTQSPSSLSASVGDRVTISCRASQDISKYLNWYQQKPGKAPKLLIYH
TSRLHSGVPSRFSGSGSGTDYTLTISSLQPEDFATYYCQQGNTLPYTFGGGTKL
EIKGGGGSGGGGSGGGGSQVQLQESGPGLVKPSETLSLTCTVSGVSLPDYGVS
WIRQPPGKGLEWLGVIWGSETTYYNSALKSRLTISKDNSKSQVFLKMSSVTAA
DTAVYYCAKHYYYGGSYAMDYWGQGTTVTVSS

The scFv composed of V_{L3} and V_{H4} was constructed into different CARs, wherein the linker between V_{L3} and V_{H4} consisted of three repeats of the GGGGS (SEQ ID NO: 11) sequence; the hinge region (SEQ ID NO: 17) and transmembrane domain (SEQ ID NO: 18) were derived from CD8 alpha; the second signaling domain was either the wild-type CD28 intracellular domain (SEQ ID NO: 19) or the CD28 intracellular domain with Lck binding site mutation (CD28Δ) (SEQ ID NO: 20); the first signaling domain was derived from the intracellular domain of CD3 zeta (SEQ ID NO: 21); and the signal peptide was derived from CD8 alpha (SEQ ID NO: 16).
> CD8 alpha signal peptide (SEQ ID NO: 16)
   MALPVTALLLPLALLLHAARP
> Hinge region (SEQ ID NO: 17)
   KPTTTPAPRPPTPAPTIASQPLSLRPEACRPAAGGAVHTRGLDFACD
> Transmembrane domain (SEQ ID NO: 18)
   IYIWAPLAGTCGVLLLSLVITLYC
>Wild-type CD28 intracellular region (SEQ ID NO: 19)
   RSKRSRLLHSDYMNMTPRRPGPTRKHYQPYAPPRDFAAYRS
> CD28 intracellular region with Lck binding site mutation (CD28Δ) (SEQ ID NO: 20)
   RSKRSRLLHSDYMNMTPRRPGPTRKHYQAYAAARDFAAYRSL
> Intracellular region of CD3 zeta (SEQ ID NO: 21)

LRVKFSRSADAPAYQQGQNQLYNELNLGRREEYDVLDKRRGRDPEMGG
KPRRKNPQEGLYNELQKDKMAEAYSEIGMKGERRRGKGHDGLYQGLSTATK
DTYDALHMQALPPR

The constructed CARs were named CAR3 (with the full-length amino acid sequence as set forth in SEQ ID NO: 22), CAR24 (with the full-length amino acid sequence as set forth in SEQ ID NO: 23), CAR1 (with the full-length amino acid sequence as set forth in SEQ ID NO: 24), and CAR2 (with the full-length amino acid sequence as set forth in SEQ ID NO: 25), respectively.
> CAR3 (SEQ ID NO: 22) 
MALPVTALLLPLALLLHAARPDIQMTQSPSSLSASVGDRVTITCRASQDIS
KYLNWYQQKPGKAPKLLIYHTSRLHSGVPSRFSGSGSGTDFTLTISSLQPEDFA
TYYCQQGNTLPYTFGGGTKLEIKGGGGSGGGGSGGGGSQVQLQESGPGLVKP
SETLSLTCTVSGVSLPDYGVSWIRQPPGKGLEWIGVIWGSETTYYNPSLKSRVT
ISVDTSKNQVSLKLSSVTAADTAVYYCAKHYYYGGSYAMDYWGQGTTVTVS
SKPTTTPAPRPPTPAPTIASQPLSLRPEACRPAAGGAVHTRGLDFACDIYIWAPL
AGTCGVLLLSLVITLYCRSKRSRLLHSDYMNMTPRRPGPTRKHYQAYAARD
FAAYRSLRVKFSRSADAPAYQQGQNQLYNELNLGRREEYDVLDKRRGRDPEM
GGKPRRKNPQEGLYNELQKDKMAEAYSEIGMKGERRRGKGHDGLYQGLSTA
TKDTYDALHMQALPPR
> CAR24 (SEQ ID NO: 23) 
MALPVTALLLPLALLLHAARPDIQMTQSPSSLSASVGDRVTITCRASQDIS
KYLNWYQQKPGKAPKLLIYHTSRLHSGVPSRFSGSGSGTDFTLTISSLQPEDFA
TYYCQQGNTLPYTFGGGTKLEIKGGGGSGGGGSGGGGSQVQLQESGPGLVKP
SETLSLTCTVSGVSLPDYGVSWIRQPPGKGLEWIGVIWGSETTYYNPSLKSRVT
ISVDTSKNQVSLKLSSVTAADTAVYYCAKHYYYGGSYAMDYWGQGTTVTVS
SKPTTTPAPRPPTPAPTIASQPLSLRPEACRPAAGGAVHTRGLDFACDIYIWAPL
AGTCGVLLLSLVITLYCRSKRSRLLHSDYMNMTPRRPGPTRKHYQPYAPPRDF
AAYRSLRVKFSRSADAPAYQQGQNQLYNELNLGRREEYDVLDKRRGRDPEM
GGKPRRKNPQEGLYNELQKDKMAEAYSEIGMKGERRRGKGHDGLYQGLSTA
TKDTYDALHMQALPPR
> CAR1 (SEQ ID NO: 24) 
MALPVTALLLPLALLLHAARPDIQMTQSPSSLSASVGDRVTISCRASQDIS
KYLNWYQQKPGKAPKLLIYHTSRLHSGVPSRFSGSGSGTDYTLTISSLQPEDFA
 
TYYCQQGNTLPYTFGGGTKLEIKGGGGSGGGGSGGGGSEVQLQESGPGLVKP
SETLSVTCTVSGVSLPDYGVSWIRQPPGKGLEWLGVIWGSETTYYNSALKSRL
TIIKDNSKSQVFLKMSSLTAADTAVYYCAKHYYYGGSYAMDYWGQGTTVTV
SSKPTTTPAPRPPTPAPTIASQPLSLRPEACRPAAGGAVHTRGLDFACDIYIWAP
LAGTCGVLLLSLVITLYCRSKRSRLLHSDYMNMTPRRPGPTRKHYQAYAAAR
DFAAYRSLRVKFSRSADAPAYQQGQNQLYNELNLGRREEYDVLDKRRGRDPE
MGGKPRRKNPQEGLYNELQKDKMAEAYSEIGMKGERRRGKGHDGLYQGLST
ATKDTYDALHMQALPPR
> CAR2 (SEQ ID NO: 25)

MALPVTALLLPLALLLHAARPDIQMTQSPSSLSASVGDRVTISCRASQDIS
KYLNWYQQKPGKAPKLLIYHTSRLHSGVPSRFSGSGSGTDYTLTISSLQPEDFA
TYYCQQGNTLPYTFGGGTKLEIKGGGGSGGGGSGGGGSQVQLQESGPGLVKP
SETLSLTCTVSGVSLPDYGVSWIRQPPGKGLEWLGVIWGSETTYYNSALKSRL
TISKDNSKSQVFLKMSSVTAADTAVYYCAKHYYYGGSYAMDYWGQGTTVTV
SSKPTTTPAPRPPTPAPTIASQPLSLRPEACRPAAGGAVHTRGLDFACDIYIWAP
LAGTCGVLLLSLVITLYCRSKRSRLLHSDYMNMTPRRPGPTRKHYQAYAAAR
DFAAYRSLRVKFSRSADAPAYQQGQNQLYNELNLGRREEYDVLDKRRGRDPE
MGGKPRRKNPQEGLYNELQKDKMAEAYSEIGMKGERRRGKGHDGLYQGLST
ATKDTYDALHMQALPPR

After constructing the four CARs shown in Figure 1 into a retroviral vector, the CAR expression plasmid, virus packaging plasmid, and viral envelope protein expression plasmid were transduced into 293T cells at a ratio of 4:3:3 using PEI transfection to produce retroviral particles. The supernatant was collected on the first and second days post-transfection and pooled as the viral harvest. Primary T cells were activated with TransAct (Miltenyi Biotec) for two days, then infected with the viral harvest. One day after infection, the virus was removed, and the T cells were cultured in RPMI 1640 medium supplemented with 10% fetal bovine serum and interleukin-2 (IL-2, 400 IU/mL). After an additional 4-7 days of culture, the CAR-positive rate was detected by staining with FITC-labeled CD19-Fc protein, and dead cells were excluded by 7-AAD staining.

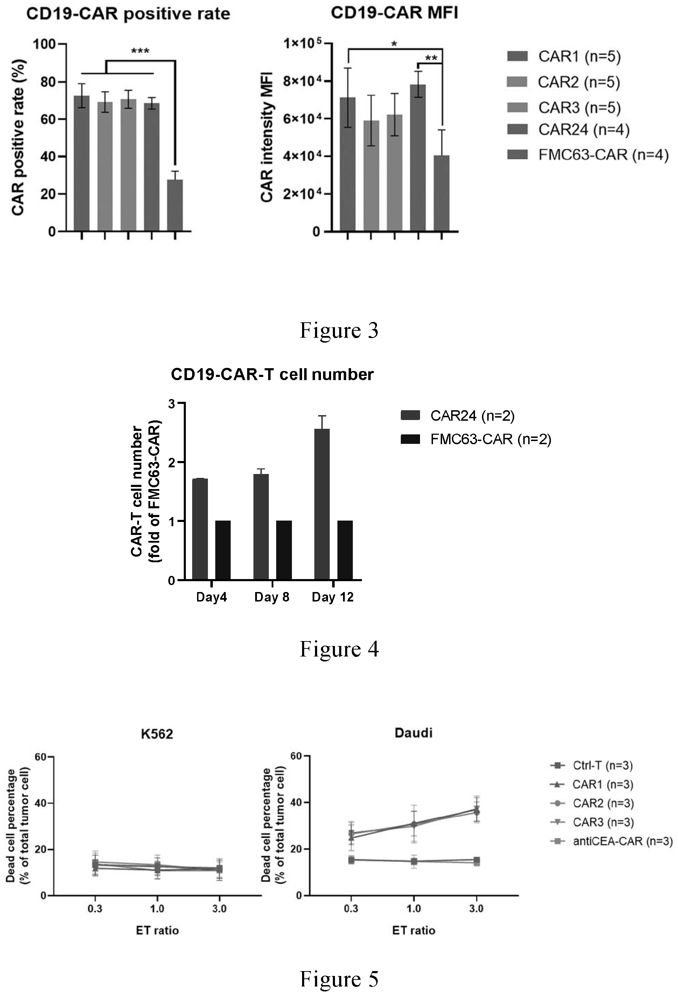

The results are shown in Figure 2, indicating that CAR1, CAR2, CAR3, and CAR24 were all normally expressed on the surface of T cells and could be recognized by the CD19 protein. This demonstrates that the designed CARs, along with the vectors and expression systems carrying them, can be applied to the preparation of CD19-targeting CAR-T cells. As shown in the statistical results of Figure 3, CAR1, CAR2, CAR3 and CAR24 all exhibited higher CAR-positive rates (2.5-2.8 fold) compared with FMC63-CAR, and their median fluorescence intensity (MFI) of FITC was also 1.6-2.1 fold higher. As shown in Figure 4, comparing the yields of CAR-T cells at 4 days, 8 days and 12 days post-infection, the yield of CAR-T cells based on the humanized scFv of the present invention was 1.7-2.7 fold higher than that of FMC63 CAR-T cells.

### Example 3: Validation of In vitro Killing Capacity of CAR-T Cells Containing Humanized scFv

The *in vitro* killing capacity of the four types of CAR-T cells prepared in Example 2 was tested.

K562 human chronic myeloid leukemia cells (CD19 surface antigen-negative) and Daudi B lymphoma cells (CD19 surface antigen-positive) were used as target cells. The K562 and Daudi cells were first labeled with CellTracker^{™} Blue (Invitrogen) for 30 minutes. After washing away the dye, they were mixed with CAR-T cells at effector-to-target (E:T) ratios of 0.3, 1, and 3, and co-cultured in X-VIVO15 medium containing 10% fetal bovine serum. Ctrl-T were mock-electroporated T cells, and their cell number in the killing assay was equivalent to that of the CAR-T group with the lowest positivity rate but the highest total T cell count among the four CAR-T groups. CAR-T cells targeting CEA (based on BW431/26 scFv) served as another control group. After 16 to 24 hours, cells were stained with 7-AAD/Annexin V-FITC, and the death proportion of K562 and Daudi cells was analyzed by flow cytometry. After gating on CellTracker^{™} Blue-positive cells, a four-quadrant plot was generated with Annexin V-FITC on the X-axis and 7-AAD on the Y-axis to determine the proportions in each quadrant. The percentage of dead cells was the sum of the three quadrants: 7-AAD⁺ Annexin V⁻, 7-AAD⁺ Annexin V⁺, and 7-AAD⁻ Annexin V⁺.

The results are shown in Figure 5, demonstrating that CAR-T cells expressing CAR1, CAR2, and CAR3 effectively killed CD19 antigen-positive Daudi B lymphoma cells but had no killing effect on CD19 antigen-negative K562 cells. This result proves that the CAR based on the scFv of the present invention can be used to prepare CAR-T cells, which exhibit good *in vitro* killing capacity and specifically target CD19 antigen-positive tumor cells.

Furthermore, the *in vitro* killing capacity of the humanized anti-CD19 CAR-T (CAR24) of the present invention was compared with that of murine-derived FMC63 anti-CD19 CAR-T. The humanized anti-CD19 CAR-T (CAR24) and the murine-derived FMC63 anti-CD19 CAR-T differ only in their scFv regions, with all other structures being identical. Daudi B lymphoma cells were used as target cells, and CAR-T targeting CEA (based on BW431/26 scFv) served as the control group. The target cells were first labeled with CellTracker^{™} Blue (Invitrogen) for 30 minutes. After washing, they were mixed with CAR-T cells at E:T ratios of 0.3, 1, and 3 and co-cultured in X-VIVO15 medium containing 10% fetal bovine serum. After 24 hours, cells were stained with 7-AAD/Annexin V-FITC, and the death proportion of K562 and Daudi cells was analyzed by flow cytometry. After gating on CellTracker^{™} Blue-positive cells, a four-quadrant plot was generated with Annexin V-FITC on the X-axis and 7-AAD on the Y-axis to determine the proportions in each quadrant. The percentage of dead cells was the sum of the three quadrants: 7-AAD⁺ Annexin V⁻, 7-AAD⁺ Annexin V⁺, and 7-AAD⁻ Annexin V⁺.

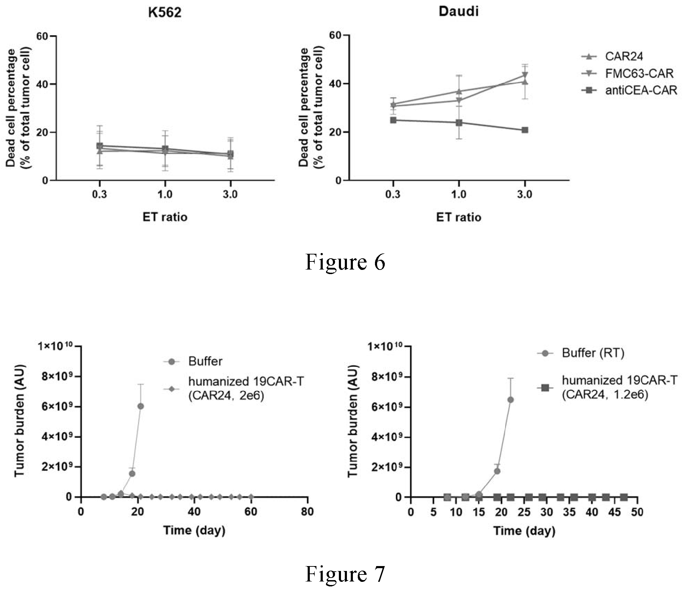

The results are shown in Figure 6, indicating that the *in vitro* killing capacity of CAR-T cells based on the humanized scFv of the present invention (CAR24) was comparable to that of CAR-T cells based on FMC63, both of which specifically killed CD19 antigen-positive tumor cells.

### Example 4: Validation of In vivo Tumor Suppression Capacity of Humanized Anti-CD19 CAR-T

Seven days (left panel of Figure 7) or five days (right panel of Figure 7) after intravenous inoculation of NOG immunodeficient mice were intravenously inoculated with 5×10⁶ Daudi B lymphoma cells carrying the Luciferase gene. The humanized anti-CD19 CAR-T (CAR24) cells prepared according to the present invention were infused seven days (left panel of Figure 7) or five days (right panel of Figure 7) after inoculation. The buffer group was injected with the formulation buffer. The luciferase activity in the mice was measured every 3-4 days after CAR-T injection. Tumor burden was represented by the total bioluminescence value (photo/s) of the mice.

The experimental results showed that humanized CD19 CAR-T exhibited good therapeutic efficacy against B lymphoma (Figure 7).

All documents mentioned in the present invention are incorporated herein by reference as if each document were individually incorporated by reference. Furthermore, it should be understood that after reading the foregoing teachings of the present invention, those skilled in the art can make various alterations or modifications to the present invention, and these equivalent forms also fall within the scope defined by the appended claims.

## Claims

1. A humanized anti-CD19 antibody, comprising an antibody heavy chain variable region as set forth in any one of SEQ ID NOs: 1-4, and an antibody light chain variable region as set forth in any one of SEQ ID NOs: 5-7.

2. The antibody of claim 1, wherein, the antibody comprises the antibody heavy chain variable region as set forth in SEQ ID NO: 1, and the antibody light chain variable region as set forth in SEQ ID NO: 5; or
the antibody comprises the antibody heavy chain variable region as set forth in SEQ ID NO: 2, and the antibody light chain variable region as set forth in SEQ ID NO: 6; or
the antibody comprises an antibody heavy chain variable region as set forth in SEQ ID NO: 3, and an antibody light chain variable region as set forth in SEQ ID NO: 6.

3. The antibody of claim 1, wherein, the structure of the antibody is shown in the following Formula A or Formula B:
V_{H}-V_{L} (A);
V_{L}-V_{H} (B)
wherein, V_{H} is the antibody heavy chain variable region; V_{L} is the antibody light chain variable region; "-" is a linker peptide or peptide bond; preferably, the structure is shown in Formula B.

4. The antibody of any one of claims 1-3, wherein, the amino acid sequence of the antibody is set forth in any one of SEQ ID NOs: 8-10.

5. A CD19-targeting antigen binding receptor (CAR), wherein, the antigen binding domain of the CAR comprises the humanized anti-CD19 antibody of claim 1.

6. The CAR of claim 5, wherein, the structure of the CAR is shown in the following Formula I:
L-scFv-H-TM-C-CS (I)
wherein,
each "-" is independently a linker peptide or a peptide bond;
L is none or a signal peptide sequence;
scFv is the antigen-binding domain targeting CD19;
H is none or a hinge region;
TM is a transmembrane domain;
C is a co-stimulatory signal molecule;
CS is an intracellular signaling sequence derived from CD3ζ.

7. The CAR of claim 6, wherein, the scFv has a structure as shown in Formula A or Formula B:
V_{H}-V_{L} (A);
V_{L}-V_{H} (B)
wherein, V_{H} is the antibody heavy chain variable region of the humanized anti-CD19 antibody; V_{L} is the antibody light chain variable region of the humanized anti-CD19 antibody; "-" is a linker peptide or peptide bond; preferably, a structure of Formula B.

8. A nucleic acid molecule encoding the humanized anti-CD19 antibody of claim 1 or the CD19-targeting CAR of claim 5.

9. A vector, wherein the vector contains the nucleic acid molecule of claim 8.

10. A host cell, which contains the vector of claim 9, or has an exogenous nucleic acid molecule of claim 8 being integrated into the chromosome, or expresses the humanized anti-CD19 antibody of claim 1 or the CD19-targeting CAR of claim 5.

11. An engineered immune cell, which contains the vector of claim 9, or has an exogenous nucleic acid molecule of claim 8 being integrated into the chromosome, or expresses the humanized anti-CD19 antibody of claim 1 or the CD19-targeting CAR of claim 5.

12. An immunoconjugate, comprising:
(a) the humanized anti-CD19 antibody of claim 1;
(b) a coupling moiety selected from the group consisting of: a detectable label, a drug, a toxin, a cytokine, a radionuclide, and an enzyme.

13. A kit comprising the humanized anti-CD19 antibody of claim 1 or the immunoconjugate of claim 12.

14. A preparation containing the humanized anti-CD19 antibody of claim 1, or the immunoconjugate of claim 12, or the engineered immune cell of claim 11, and a pharmaceutically acceptable carrier.

15. Use of the humanized anti-CD19 antibody of claim 1, or the engineered immune cell of claim 11, or the immunoconjugate of claim 12 for (1) preparing a drug or preparation for treating cancer or tumors; (2) preparing a reagent or kit for detecting CD19 molecules.
